# EUROPEAN PATENT APPLICATION

(11) **EP 4 019 592 A1**
(43) Date of publication of application: **29.06.2022**
(21) Application number: 20306686.5
(22) Date of filing: 23.12.2020
(51) Int. Cl.: C08L 93/04, C09J 153/02, C09J 193/04, A61F 13/00

(54) **HOT MELT ADHESIVE COMPOSITION**

(71) Applicant: Bostik SA, 92700 Colombes (FR)
(72) Inventor: BELLINI, Clement, 60280 VENETTE (FR)
(74) Representative: Arkema Patent

(57) **Abstract**

The present invention relates to a hot melt adhesive composition comprising: at least one styrene block copolymer; at least one bio-based tackifier resin; and optionally at least one plasticizer chosen from: an esterified fatty acid; and a stand oil; wherein the hot melt adhesive composition has a glass transition temperature higher than 0°C.

The present invention further relates to the use of said hot-melt adhesive composition for bonding two substrates and to an article comprising said hot-melt adhesive composition.

## Description

### Technical field

The present invention relates to a bio-based hot melt adhesive composition. The present invention also relates to articles made using this hot melt adhesive composition.

### Technical background

Adhesives are often used to bond substrates together so as to maintain the two substrates in a fixed relation to each other. In the area of industrial adhesives, hot melt adhesives are commonly used to bond together a wide variety of articles including disposable absorbent articles comprising non-woven substrates such as adult incontinence products, disposable diapers, sanitary napkins, bed pads, puppy pads, medical dressings, etc.

There can be multiple hot melt adhesives used in the manufacture of a disposable absorbent article. For example, in the manufacture of a disposable diaper, adhesives are used in construction (for example for bonding the backsheet to the nonwoven and optionally the absorbent pad), elastic attachment (for example for bonding the elastic material to the backsheet in for example the leg or waist area), and for the core stabilization (for example for applying an adhesive to the absorbent core to increase the strength of the core).

Hot melts are generally understood to be room temperature-solid, water and solvent-free adhesives that are applied from the melt onto the parts to be glued together and after assembly physically set by solidifying on cooling.

Conventional hot melt adhesives are petrochemical-based, and rely on energy-intensive processes. In addition, a large percentage of the petroleum is transported from various parts of the world, which increases the carbon footprint. Generally, zero to very few raw materials in hot melt adhesives are made from renewable resources and thus contribution is not enough on the impact of the environment.

There is an increased desire to reduce carbon footprint and to produce environmentally sound products. One method of making an environmentally sound adhesive is to decrease carbon footprint by forming a hot melt adhesive from materials having a high renewable resource content.

However, adhesives made from materials having a high renewable resource content are known to be more unstable to air oxidation compared to petroleum-based adhesives. Therefore, thermal stability is a key parameter for the development of such adhesives.

Document WO 2020/092515 relates to a high bio-based content pressure sensitive hot melt adhesive composition comprising plasticizers made from renewable-based feedstock.

Document WO 99/13016 relates to a process for bonding tissue or nonwoven to similar or dissimilar substrates comprising a step of applying a molten hot melt adhesive composition to at least one substrate, said composition containing, as the plasticizing agent, a hydrogenated or non-hydrogenated natural oil selected from the group consisting of fatty acids containing about 6 to 22 carbon atoms.

Document EP 1847579 relates to a hot melt adhesive having a low viscosity and a high melting point, with a long open time. Such adhesive comprises a polymer, a tackifier, and a saturated fatty acid as the main or sole wax component.

Document EP 2371922 relates to a hot melt adhesive comprising from 3 to 35 % by weight of one or more styrene block copolymers, more than 25 % and less than 60 %, by weight, of triglycerides of fatty acids with an average carbon chain length distribution from C14 to C20 and a iodine value lower than 50; and from 5 to 70 % by weight of a tackifier or blend of tackifiers selected from terpenic tackifiers, rosin tackifiers and rosin ester tackifiers.

Document EP 2452979 relates to the use of thermoplastic oil-modified alkyd resins as the predominant component of a hot melt adhesive which can be employed for instance as a construction adhesive in the manufacture of disposable articles.

Document US 2016/0032156 relates to a pressure-sensitive adhesive comprising at least one synthetic rubber based on at least one vinyl aromatic compound and at least one diene; the proportion of synthetic rubber being at most 45 % by weight; at least one bio-based adhesive resin based to an extent of at least 85 % on renewable raw materials and making up a proportion of at least 1 % by weight to at most 25 % by weight of the entire composition of the pressure-sensitive adhesive.

There is thus a need for providing environment-friendly hot melt adhesives that present a good adhesion, even at high temperatures, while maintaining good physicochemical properties.

### Summary of the invention

It is a first object of the invention to provide a hot melt adhesive composition comprising:
- at least one styrene block copolymer;
- at least one bio-based tackifier resin, and
- optionally at least one plasticizer chosen from:
   - an esterified fatty acid; and
   - a stand oil;
   wherein the hot melt adhesive composition has a glass transition temperature higher than 0°C.

According to some embodiments, the styrene block copolymer is chosen from a styrene-isoprene-styrene triblock copolymer and/or a styrene-butadiene-styrene triblock copolymer.

According to some embodiments, the styrene block copolymer comprises at least one polydiene block which is fully or partly made from renewable materials, and is preferably chosen from a polybutadiene block, a polyfarnesene block and a polymyrcene block.

According to some embodiments, the tackifier resin is chosen from a terpene derivative and/or a rosin derivative.

According to some embodiments, the tackifier resin is a terpene derivative chosen from a polyterpene homopolymer, a copolymer of terpene with a diene monomer, and a phenolic-modified terpene resin.

According to some embodiments, the tackifier resin is a rosin derivative chosen from a rosin and a rosin ester, preferably from a fully hydrogenated rosin and a fully hydrogenated rosin ester, and more preferably wherein the rosin derivative is a fully hydrogenated rosin ester.

According to some embodiments, the esterified fatty acid results from a transesterification, reaction between a triglyceride and a mono-alcohol or a polyol.

According to some embodiments, the fatty acid comprises from 6 to 30 carbon atoms and is chosen from a hydrogenated or a non-hydrogenated fatty acid.

According to some embodiments, the stand oil of vegetable oil is obtained by heating said vegetable oil in the absence of oxygen and at a temperature higher than 200°C, and preferably higher than 270°C.

According to some embodiments, the stand oil is a stand oil of a vegetable oil comprising fatty acids containing from 6 to 30 carbon atoms.

According to some embodiments, the composition has a biocarbon content equal to or higher than 70 %.

According to some embodiments, the composition comprises from 10 to 40 % by weight of the at least one styrene block copolymer relative to the total weight of the composition, and/or from 20 to 80 % by weight of the at least one tackifier resin relative to the total weight of the composition, and/or from 0 to 25 % by weight of the at least one plasticizer relative to the total weight of the composition.

According to some embodiments, the tackifier resin has a Ring and Ball softening point from 70 to 150°C.

The invention also relates to the use of above hot-melt adhesive composition for bonding two substrates.

The invention also relates to an article comprising the hot melt adhesive composition described above.

According to some embodiments, the article is a disposable absorbent article chosen from disposable diapers, adult incontinence products, sanitary napkins, medical dressings, bandages, surgical pads, and pet training pads.

The present invention makes it possible to meet the abovementioned need. In particular, the invention provides environment-friendly hot melt adhesives that present a good adhesion, even at high temperatures, while maintaining good physicochemical properties.

This is achieved by the composition of the present invention. More particularly, on the one hand the hot melt adhesive composition of the present invention is prepared at least partly from renewable resources which results in a composition having a high biocarbon content and thus being environment-friendly. By *"environment-friendly adhesive"* is meant an adhesive which has a biocarbon content of at least 70%. On the other hand, the hot melt adhesive composition of the present invention, notably due to a glass transition temperature equal to or higher than 0°C, presents good coater processability and adhesion properties.

Advantageously, the present invention makes it possible to provide environment-friendly hot melt adhesives that also present a good thermal stability.

### Detailed description

The invention will now be described in more detail without limitation in the following description.

The carbon of a biomaterial derives from the photosynthesis of plants and therefore from atmospheric CO₂. The degradation (by degradation is also meant combustion/incineration at the end of their life) of these materials into CO₂ does not therefore contribute to warming since there is no increase in the carbon emitted into the atmosphere. The CO₂ balance of biomaterials is therefore clearly improved and contributes to reducing the carbon footprint of the products obtained (only the energy for manufacturing is to be taken into account). On the contrary, a material of fossil origin which also degrades into CO₂ will contribute to an increase in the level of CO₂ and therefore to global warming.

The components of the invention will therefore have a carbon footprint which will be better than that of compounds obtained from a fossil source.

The term "*bio-carbon*" indicates that the carbon is of renewable origin, or of natural origin and originates from a biomaterial, as indicated below. The biocarbon content and the biomaterial content are expressions denoting the same value.

A material of renewable origin, also called biomaterial, is an organic material in which the carbon derives from CO₂ recently fixed (on a human scale) by photosynthesis from the atmosphere. On earth, this CO₂ is captured or fixed by plants. At sea, CO₂ is captured or fixed by bacteria or plankton carrying out photosynthesis. A biomaterial (100% carbon of natural origin) has a ¹⁴C/¹²C isotope ratio greater than 10⁻¹², typically of approximately 1.2 x 10⁻¹², while a fossil material has a zero ratio. Indeed, the isotope ¹⁴C is formed in the atmosphere and is then integrated by photosynthesis, according to a time scale of a few decades at most. The half-life of ¹⁴C is 5730 years. Thus, materials resulting from photosynthesis, namely plants in general, necessarily have a maximum ¹⁴C isotope content.

The biomaterial content or biocarbon content is determined by using the standards ASTM D 6866 (ASTM D 6866-06) and ASTM D 7026 (ASTM D 7026-04). The ASTM D 6866 standard is "Determining the Biobased Content of Natural Range Materials Using Radiocarbon and Isotope Ratio Mass Spectrometry Analysis*",* while the ASTM D 7026 standard is "Sampling and Reporting of Results for Determination of Biobased Content of Materials via Carbon Isotope Analysis*".* The second standard makes reference in its first paragraph to the first standard.

The first standard describes a test for measuring the ¹⁴C/¹²C ratio of a sample and compares it with the ¹⁴C/¹²C ratio of a reference sample of 100 % renewable origin, to give a relative percentage of C of renewable origin in sample. The standard is based on the same concepts as ¹⁴C dating, but without applying the dating equations.

The ratio thus calculated is referred to as the *"pMC"* (percent Modern Carbon). If the material to be analyzed is a mixture of biomaterial and fossil material (without radioactive isotope), then the pMC value obtained is directly correlated with the quantity of biomaterial present in the sample. The reference value used for the ¹⁴C dating is a value dating from the 1950s. This year was chosen because of the existence of nuclear tests in the atmosphere which introduced large quantities of isotopes into the atmosphere after this date. The 1950 reference corresponds to a pMC value of 100. Taking into account the thermonuclear tests, the current value to be retained is approximately 107.5 (which corresponds to a correction factor of 0.93). The radioactive carbon signature of a current plant is therefore 107.5. A signature of 54 pMC and 99 pMC therefore correspond to an amount of biocarbon in the sample of 50 % and 93 %, respectively.

### Hot melt adhesive composition

The invention relates to a hot melt adhesive composition which has a high biocarbon content as explained above.

By *"hot melt*" is meant that the adhesive composition requires to be heated at least at 120°C, preferably at least at 140°C, to be applied on a substrate. The hot melt adhesive composition is thus solid at 23°C.

First of all, the hot melt adhesive composition comprises at least one styrene block copolymer.

The styrene block copolymer according the invention may be chosen from a linear diblock copolymer having an AB structure (in other words a copolymer comprising one terminal styrene block and one block formed from at least one type of monomers other than styrene monomers), a linear triblock copolymer having an ABA structure (in other words a copolymer comprising two terminal styrene blocks and a mid-chain block formed from at least one type of monomers other than styrene monomers), a radial block copolymer having the (AB)ₙY structure, and mixtures of such copolymers.

A represents a non-elastomeric polystyrene block.

B represents an elastomeric block polymer. B may notably be a polydiene, e.g. polybutadiene and/or polyisoprene block.

Y represents a multivalent compound.

Finally, n is an integer which is equal to at least 3.

According to some embodiments, the elastomeric block B can be post treated through partial or total hydrogenation to improve its heat stability.

Preferably, the styrene block copolymer useful according the invention is chosen from the following linear triblock copolymers:
- styrene-butadiene-styrene copolymer (SBS) with or without styrene-butadiene diblock (SB),
- styrene-isoprene-styrene copolymer (SIS) with or without styrene-isoprene diblock (SI),
- styrene-ethylene-butylene-styrene copolymer (SEBS),
- styrene-propylene-butylene-styrene copolymer (SPBS),
- styrene-butadiene-butylene-styrene copolymer (SBBS),
- styrene-ethylene-propylene-styrene copolymer (SEPS),
- styrene-ethylene-ethylene-propylene-styrene copolymer (SEEPS),
- styrene-isoprene-butadiene-styrene copolymer (SIBS),
- styrene-isoprene-propylene-styrene copolymer (SIPS),
- and any mixture thereof.

More preferably, the styrene block copolymer is a linear triblock copolymer having an ABA structure, as defined above, and even more preferably a linear SIS triblock copolymer.

When the styrene block copolymer is a mixture of linear triblock copolymer having an ABA structure and linear diblock copolymer having an AB structure, as defined above, the linear diblock content preferably range from 1 to 60% by weight relative to the total weight of the triblock and diblock mixture.

The amount of the end blocks A in the linear triblock copolymer of ABA structure, as defined above, may range from 14 to 51 % by weight, preferably from 20 to 40 % by weight, relative to the total weight of the linear triblock copolymer of ABA structure or, in the case of a mixture of linear triblock and diblock copolymers of ABA and AB structures, relative to the total weight of the triblock and diblock mixture.

Useful commercial styrene block copolymers include KRATON D and G ^{®} series from Kraton Polymers, EUROPRENE Sol T ^{®} series from Versalis (Eni group), SOLPRENE ^{®} series from Dynasol Elastomers, SINOPEC ^{®} series from SINOPEC and Taipol ^{®} and Vector^{®} series from TSRC Corporation.

As examples of useful styrene block copolymers, mention may be made of:
- Sinopec^{®} YH-1126, a linear SIS triblock copolymer with a styrene content of 16 %, and a diblock content of 50 %;
- Sinopec^{®} YH-1206, a linear SIS triblock copolymer with a styrene content of 30 %, and a diblock content of less than 1 %;
- Kraton^{®} D1152, a mixture of linear SBS triblock and SB diblock copolymers, with a styrene content of 29.5 % by weight relative to the total weight of the mixture, an average molecular weight of around 122 000 g/mol, a melt flow index or MFI (measured according to ISO1133) of 8.5 grams/ 10 minutes at 200°C under a load of 5 kilograms, and a SB diblock content of around 17 % by weight relative to the total weight of the mixture.

- Kraton^{®} D1161, a mixture of linear SIS triblock and SI diblock copolymers, with a styrene content of 15 % by weight relative to the total weight of the mixture, an MFI (measured according to ISO1133) of 9 g/10 min at 200°C under a load of 5 kg, an average molecular weight of around 220 000 g/mol, and a SI diblock content of around 19 % by weight relative to the total weight of the mixture.
- Taipol^{®} SBS 4202 from TSRC Corporation, a linear SBS triblock copolymer with a styrene content of 40 % by weight relative to the total weight of the triblock copolymer, an MFI (measured according to ASTM D1238) of 3 to 10 g/10 min at 190°C under a load of 5 kg, an average molecular weight of around 102 400 g/mol.
- Vector^{®} 4411 from TSRC Corporation, a linear SIS triblock copolymer with a styrene content of 44 % by weight relative to the total weight of the triblock copolymer, an MFI (measured according to ASTM D1238) of 40 g/10 min at 200°C under a load of 5 kg, an average molecular weight of around 106 000 g/mol.

The hot melt adhesive composition may comprise from 10 to 40 %, and preferably from 15 to 30 % by weight of the at least one styrene block copolymer (or of the mixture of styrene block copolymers in case a plurality of styrene block copolymers are present in the hot melt adhesive composition) relative to the total weight of the composition.

According to some embodiments, the elastomeric block of the styrene block copolymer is bio-based in other words derives from a biomaterial (as explained above). In this case, the styrene block copolymer may have a biocarbon content equal to or higher than 75 %, and preferably equal to or higher than 80 %. Thus, the styrene block copolymer may have a ¹⁴C/¹²C isotope ratio equal to or higher than 0.9 x 10⁻¹², and preferably equal to or higher than 0.96 x 10⁻¹². For example, the elastomeric block polymer may be chosen from a polybutadiene block a polymyrcene block, a polyfarnesene block and mixtures thereof.

The hot melt adhesive composition further comprises at least one tackifier resin chosen from bio-based resins such as a terpene derivative and a rosin derivative. By *"bio-based tackifier resin"* is meant a tackifier resin that derives at least partially from a biomaterial. Thus, such resin may have a biocarbon content equal to or higher than 85 %, preferably equal to or higher than 90 %, and more preferably equal to or higher than 93 %. According to some embodiments, such resins may have a biocarbon content of 100 %. Therefore, these resins may have a ¹⁴C/¹²C isotope ratio equal to or higher than 1.02 x 10⁻¹², preferably equal to or higher than 1.08 x 10⁻¹², more preferably equal to or higher than 1.11 x 10⁻¹², or even a ¹⁴C/¹²C isotope ratio of 1.2 x 10⁻¹².

In case the tackifier resin is a terpene derivative, it may be chosen from a polyterpene homopolymer, a copolymer of terpene with a diene monomer, and a phenolic-modified terpene resin.

In case the tackifier resin is a rosin derivative, it may be chosen from a rosin and a rosin ester, preferably from a partly or fully hydrogenated rosin and a partly or fully hydrogenated rosin ester, and more preferably from a fully hydrogenated rosin and a fully hydrogenated rosin ester.

More particularly, the tackifier resin according to the present invention may be chosen from the following classes:
- natural and modified rosins such as, for example, gum rosins, wood rosins, tall-oil rosins, distilled rosins, hydrogenated rosins, dimerized rosins and polymerized rosins;
- glycol, glycerol and pentaerythritol esters of natural and modified rosins, such as, for example, the glycerol esters of pale wood rosin, the glycerol esters of hydrogenated rosin, the glycerol esters of polymerized rosin, the pentaerythritol esters of pale wood rosin, the pentaerythritol esters of hydrogenated rosin, the pentaerythritol esters of tall oil rosin and the phenolic modified pentaerythritol esters of rosin;
- polyterpene resins include hydrogenated polyterpene resins having a Ring and Ball softening point of from about 20°C to 150°C, the latter polyterpene resins generally resulting from the polymerization of terpene hydrocarbons, such as the monoterpene known as pinene, in the presence of Friedel-Crafts catalysts at moderately low temperatures;
- styrenic-modified terpene resins and phenolic-modified terpene resins such as, for example, those resulting from the condensation, in an acidic medium, of a terpene and a phenol.

As examples of commercially available tackifier resins belonging to the first class described above, mention may be made of:
- unmodified natural tall oil rosins from Arizona Chemical Company sold under the trade names Sylvaros^{®} (85, 90 and NCY),
- the partially hydrogenated rosin from Eastman sold under the trade name Foralyn^{®} E;
- the fully hydrogenated rosin from Eastman sold under the trade name Foral^{®} AX-E;
- the fully hydrogenated rosin from DRT sold under the trade name Foral^{®}DX;
- the fully hydrogenated rosin ester from DRT sold under the trade name Foral^{®} 105,
- the dimerized rosin from Eastman sold under the trade name Dymerex^{®}.

As examples of commercially available tackifier resins belonging to the second class described above, mention may be made of the resins available from Arizona Chemical Company such as:
- Sylvalite^{®} RE 100L, a pentaerythritol based tall-oil rosin ester, and
- Sylvalite^{®} RE 85L, a glycerol ester of tall oil rosin.

As examples of commercially available tackifier resins belonging to the third class described above, mention may be made of:
- the polyterpene tackifiers from Arizona Chemical company sold under the trade names Sylvagum^{®} TR and Sylvares^{®} TR series (7115, 7125, A25L, B115, M1115);
- the polyterpene from DRT sold under the trade name DERCOLYTE^{®}M105, resulting from the polymerization of α-pinene and β-pinene and having a Ring and Ball softening point of 105°C;
- Biofene ^{®} from TOTAL which is a polyfarnesene resin.

As examples of commercially available tackifier resins belonging to the fourth class described above, mention may be made of:
- the terpene phenol resins from the Arizona Chemical Company sold under the trade names Sylvares^{®} TP (96, 2040, 300, 7042, 2019).

The tackifier rosin derivate may have a solid or a liquid form.

In addition, according to preferred embodiments, the tackifier resin according to the present invention may have a Ring and Ball softening point from 70 to 150°C. The softening point may be measured by using the ASTM E28 standard.

The hot melt adhesive composition may comprise from 20 to 80 %, and preferably from 45 to 65 % by weight of the tackifier resin (or of the mixture of tackifier resins in case a plurality of tackifier resins are present in the hot melt adhesive composition) relative to the total weight of the composition.

The hot melt adhesive composition may further comprise at least one plasticizer chosen from an esterified fatty acid and a stand oil of a vegetable oil.

In case the plasticizer is an esterified fatty acid, the fatty acid may comprise from 6 to 30 carbon atoms, preferably from 10 to 22 carbon atoms, more preferably from 14 to 22 carbon atoms and even more preferably from 16 to 18 carbon atoms.

The fatty acid may be derived from a vegetable oil such as castor oil, sunflower oil, coconut oil, corn oil, canola oil, olive oil, palm oil, cottonseed oil, rapeseed oil, safflower oil, soybean oil, sesame oil, olive oil, almond oil, avocado oil, hemp oil, and linseed oil.

A vegetable oil is a composition comprising triple esters of fatty acids and of glycerol (also called *"triglycerides"*).

The expression *"fatty acids derived from vegetable oil*" is thus intended to designate the fatty acids present in the molecular structure of these triglycerides. Said fatty acids can be obtained, in the form of isolated compounds, by saponification, hydrolysis or methanolysis of said triglycerides.

The fatty acid may thus be chosen from myristic acid, palmitic acid, stearic acid, oleic acid, linoleic acid, and ricinoleic acid.

The fatty acid may be a hydrogenated or non-hydrogenated fatty acid.

According to some embodiments, the esterified fatty acid may result from the transesterification reaction between a mono-alcohol and the triglyceride. In this case, the mono-alcohol may notably be chosen from methanol, ethanol, propanol, isopropanol, butanol, and 2-ethylhexanol.

In addition, according to preferred embodiments, the alcohol may derive from a biomaterial which makes it thus possible to increase the biocarbon content of the plasticizer and thus of the composition.

An example of an esterified fatty acid resulting from the transesterification reaction between a mono-alcohol and the triglyceride may be the commercially available RADIA 7916 commercialized by OLEON.

According to other embodiments, the esterified fatty acid may result from a transesterification reaction between a polyol and the triglyceride. In this case, the polyol may be chosen from glycerol and pentaerythritol.

An example of an esterified fatty acid resulting from a transesterification reaction between a polyol and the triglyceride may be the commercially available PIONIER^{®} TP130S from H&R which is a hydrogenated esterified soybean oil and the commercially available RADIA 7176 (Pentaerythritol Tetrastearate) commercialized by OLEON.

The esterified fatty acid may also be an oligoester of castor oil (the side OH groups of the castor oil being transesterified with the ester functions of the castor oil itself, thus eliminating glycerol), such as VEOPUR 759035 commercialized by VANDEPUTTE.

According to other embodiments, as mentioned above, the plasticizer is a stand oil. By *"stand oil*" is meant a polymerized vegetable oil.

The stand oil is obtained by heating said vegetable oil in the absence of oxygen and at a temperature higher than 200°C, preferably higher than 270°C and even more preferably at a temperature from 270 to 360°C.

It is generally accepted that this heating causes a thermal polymerization reaction of the oil, which involves in particular the polymerization of the double bonds and the crosslinking of the corresponding triglycerides, which has the effect of increasing the viscosity of said oil. This reaction is often referred to as a *"standolization reaction".*

The heating of the vegetable oil is maintained for a time sufficient to obtain a Brookfield viscosity, measured at 20°C, greater than or equal to 50 mPa.s, preferably greater than or equal to 100 mPa.s.

According to some embodiments, the stand oil is a stand oil of a vegetable oil whose fatty acids contain 6 to 30 carbon atoms.

According to preferred embodiments, the stand oil is a stand oil of a vegetable oil whose derived fatty acids comprise a proportion of at least 75 % of fatty acids comprising from 16 to 22 carbon atoms, said proportion being expressed as a percentage in weight based on the total weight of fatty acids derived from said oil.

According to preferred embodiments, the proportion of fatty acids comprising from 16 to 22 carbon atoms may be at least 80 % by weight, and even more preferably at least 85 % by weight, relative to the total weight of the fatty acids derived from vegetable oil.

The stand oil may be chosen from stand oil obtained from sunflower oil, rapeseed oil, linseed oil and soybean oil.

According to some embodiments, the stand oil is stand oil obtained from soybean oil, the Brookfield viscosity of which, measured at 20°C, may be greater than or equal to 200 mPa.s, preferably from 0.2 to 9 Pa.s, and more preferably from 0.2 to 5 Pa.s. This viscosity is measured by using the ASTM method D-3236.

According other embodiments, the stand oil is a stand oil of linseed oil, the Brookfield viscosity of which, measured at 20°C, may be greater than or equal to 100 mPa.s, preferably from 0.1 to 60 Pa.s, and more preferably from 2 to 10 Pa.s. This viscosity is measured by using the ASTM method D-3236.

Stand oils are commercially available, and we can cite for example the following products available from the company Vandeputte:
- as soybean stand oil, Veopol^{®} 315002 whose Brookfield viscosity at 20°C is 267 mPa.s and Veopol^{®} 215035 whose Brookfield viscosity at 20°C is 4420 mPa.s;
- as flax stand oil, Veopol^{®} 212055 whose Brookfield viscosity at 20°C is of 5870 mPa.s.

According to some embodiments, only one plasticizer is present in the hot melt adhesive composition of the present invention.

According to other embodiments, more than one (for example two, or three, or four) plasticizers are present in the hot melt adhesive composition of the present invention.

The adhesive composition of the present invention may comprise from 0 to 25 %, and preferably from 7 to 22 % by weight of the at least one plasticizer (or of the mixture of plasticizers) relative to the total weight of the composition.

According to some embodiments, the adhesive composition of the present invention is devoid of a plasticizer. In this case, the tackifier resin described above itself may serve as a plasticizer.

However, according to preferred embodiments, the adhesive composition comprises a plasticizer in addition to the tackifier resin.

The plasticizer according to the present invention derives from a biomaterial. Thus, such plasticizer may have a biocarbon content equal to or higher than 80 %, and preferably equal to or higher than 85 %. According to some embodiments, such plasticizer may have a biocarbon content of equal to or higher than 95 %, or even 100 %. Therefore, the plasticizer may have a ¹⁴C/¹²C isotope ratio equal to or higher than 0.96 x 10⁻¹², preferably equal to or higher than 1.02 x 10⁻¹², more preferably equal to or higher than 1.08 x 10⁻¹², more preferably equal to or higher than 1.14 x 10⁻¹², or even a ¹⁴C/¹²C isotope ratio equal to 1.2 x 10⁻¹².

The hot melt adhesive composition of the invention may optionally include additives. Such additives may include waxes, antioxidants, pigments, brightening agents, colorants, fluorescing agents, dyestuffs, fillers, flow and leveling agents, wetting agents, surfactants, antifoaming agents, rheology modifiers, emulsifiers, humectants, gelling agent, colorants, other surface modifying agents, fragrances, and permeation enhancers. Preferably, the additives are also bio-based. Additives may be incorporated in minor amounts, for example up to about 10 %, preferably up to 5 %, by weight, into the hot melt adhesive composition of the present invention.

Useful antioxidants may include for example, pentaerythritol tetrakis[3,(3,5-di-tert-butyl-4-hydroxyphenyl)propionate], 2,2'-methylene bis(4-methyl-6-tertbutylphenol), phosphites including, e.g., tris-(p-nonylphenyl)-phosphite (TNPP) and bis(2,4-di-tert-butylphenyl)4,4'-diphenylene-diphosphonite, di-stearyl-3,3'-thiodipropionate (DSTDP), and combinations thereof.

Useful antioxidants are commercially available under a variety of trade names including, e.g., the IRGANOX series of trade names including IRGANOX 1010, IRGANOX 565, and IRGANOX 1076 hindered phenolic antioxidants and IRGAFOS 168 phosphite antioxidant, all of which are available from BASF Corporation, and ETHYL 702 4,4'-methylene bis(2,6-di-tert-butylphenol).

The hot melt adhesive composition of the present invention has a high biocarbon content and contains a low amount of petroleum-based components. The biocarbon content of the adhesive is greater than or equal to 70 %, preferably greater than or equal to 75 %, and more preferably greater than or equal to 80 %. Thus, the hot melt adhesive composition may have a ¹⁴C/¹²C isotope ratio equal to or higher than 0.84 x 10⁻¹², preferably equal to or higher than 0.9 x 10⁻¹², more preferably equal to or higher than 0.96 x 10⁻¹².

The hot melt adhesive composition has a glass transition temperature higher than 0°C. This makes it possible to obtain compositions having at the same time a high biocarbon content and a good adhesion. The glass transition temperature may be from 0 to 1°C; or from 1 to 2°C; or from 2 to 4°C; or from 4 to 6°C; or from 6 to 8°C; or from 8 to 10°C; or from 10 to 12°C; or from 12 to 14°C; or from 14 to 16°C; or from 16 to 18°C; or from 18 to 20°C. The glass transition temperature is measured by dynamic mechanical analysis (DMA).

In addition, the hot melt adhesive composition may have a viscosity at 149°C from 900 to 15000 mPa.s, and preferably from 900 to 13000 mPa.s. This viscosity is measured by using the ASTM method D-3236. The hot melt adhesive composition of the present invention may further have a rheological softening temperature from 40 to 150°C, and preferably from 60 to 130°C The rheological softening temperature is measured according to the method entitled "Dynamical Mechanical Analysis (DMA) - Temperature Sweep" described below.

The hot melt adhesive composition of the present invention makes it possible to achieve an average peel strength higher than 1 N/inch, and preferably higher than 1.25 N/inch with an adhesive coating weight of 3 gsm. The average peel strength is measured according to the method entitled "Peel measured initially at 23 °C" described below.

The composition of the present invention may be prepared by mixing the styrene block copolymer with the tackifier resin, the plasticizer and any other additional compounds and then heating this mixture at a temperature from 100 to 180 °C and for a duration from 1 to 8 hours.

Alternatively, the composition of the present invention may be prepared by heating a mixture formed from the styrene block copolymer, the plasticizer and any other additional compounds at a temperature from 100 to 180 °C and for a duration from 1 to 8 hours and then adding the tackifier resin.

The composition may then be cooled at a temperature from 20 to 30°C, and preferably at a temperature around 23°C (ambient temperature).

### Use

The invention also relates to the use of the hot-melt adhesive composition as described above, for bonding two substrates.

In this case, the process of manufacturing an assembly product (or laminate) may comprise:
- a step (i) of heating the hot-melt adhesive composition according to the invention, e.g. at a temperature ranging from 130°C to 180°C, for at least a period of time long enough to render the hot melt adhesive composition liquid enough to be applied on a substrate (for example at least two hours), then
- a step (ii) of coating said composition on a first substrate, then
- a step (iii) of putting into contact the coated surface of the first substrate with the surface of a second substrate, so as to form an adhesive joint bonding the two substrates.

The substrates may be different or of same nature, with various forms (layer or film, strands, fluff).

Preferably, each substrate may be chosen independently from one another among films (such as polyolefin films (polyethylene and/or polypropylene films)), release liners, porous substrates, cellulose substrates, sheets (such as paper, and fiber sheets), paper products, woven and nonwoven webs, fibers (such as synthetic polymer fibers such as nylon, rayon, polyesters, acrylics, polypropylenes, polyethylene, polyvinyl chloride, polyurethane, and natural cellulose fibers such as wood pulp, cotton, silk and wool), glass and tape backings.

The composition according to the invention may be coated or applied with a variety of application techniques known in the art, which include contact type application (such as slot die coating) and non-contact type application (such as spraying or fiberization).

Therefore, the hot melt adhesive composition may be used in a variety of applications and constructions including disposable absorbent articles such as disposable diapers, adult incontinence products, sanitary napkins, medical dressings (such as wound care products) bandages, surgical pads, pet training pads (such as puppy pads) and meat-packing products and components of absorbent articles including an absorbent element, absorbent cores, impermeable layers (such as backsheets), tissue (such as wrapping tissue), acquisition layers and woven and nonwoven web layers (such as top sheets, absorbent tissue) and elastics.

The hot melt adhesive composition is preferably used as a construction adhesive in a disposable absorbent article.

In a typical application for the manufacture of a disposable absorbent article, a body fluid impermeable backsheet may be bonded to a nonwoven substrate. The adhesive composition of the present invention may also be used to bond at least one additional layer or material selected from the group consisting of absorbents, tissues, elastomeric materials, superabsorbent polymers, and combinations thereof. The body fluid impermeable backsheet is typically a polyolefin film (such as polyethylene, polypropylene, ethylene vinyl acetate, ethylene copolymer).

The hot melt adhesive composition can be used to contain and/or provide strength to the absorbent core of a disposable absorbent article (such as as a core stabilization adhesive). The absorbent core can include many different materials including natural cellulose fibers (such as wood pulp, fibers, cotton, fluff) and superabsorbent polymers (such as polyacrylates).

### Examples

The following examples illustrate the invention without limiting it.

### Brookfield viscosity:

The Brookfield viscosity was measured according to the standard method ASTM D-3236, using a Brookfield viscosimeter of the type Spindle 27, at a temperature of about 163°C.

### Peel measured initially at 23°C:

A rectangular strip measuring 25 mm by approximately 10 cm was cut out from the laminate prepared below in the coated central area of the laminate.

The two individual substrates were separated, starting from one end of the above rectangular strip (as a test specimen) and over approximately 2 cm.

The two free ends thus obtained were fixed to two clamping devices respectively connected to a stationary part and a movable part of a tensile testing device which are located on a vertical axis.

While a drive mechanism communicates, to the movable part, a uniform speed of 300 mm/minute, resulting in the separation of the two substrates, the separated ends of which were gradually displaced along a vertical axis while forming an angle of 180°, the stationary part, connected to a dynamometer, measured the force withstood by the test specimen thus held.

The result corresponding to the peel after 24 hours at 23°C, is expressed in N/25 mm.

### Dynamical Mechanical Analysis (DMA) - Temperature Sweep

A rheometer applied a shear stress to an adhesive and measured the resulting strain (shear deformation) response at constant temperature.

Equipment: MCR 301 Rheometer by Anton Paars, which applied a small oscillatory stress to achieve a constant oscillatory strain within the linear viscoelastic region of the adhesive (e.g. 1%).

Temperature-dependent parameters:
- Storage Modulus
- Loss Modulus
- Loss Factor

General protocol: The adhesive was placed between a Peltier plate acting as lower, fixed plate and an upper plate with a radius R of 10 mm, which was connected to the drive shaft of a motor to generate the shear stress. The gap between both plates has a height H of 1,500 µm. The Peltier plate controls the temperature of the material (+/- 0.5°C).

Test execution:
- After GAP 0 at 80°C, setting the temperature to the start temperature (*i.e.* 150°C);
- placing the adhesive (1 cm³) on the Peltier plate;
- lowering the upper plate to a gap of 1,700 µm till its contacts the adhesive;
- removing excessive material and setting the temperature to 120°C;
- lowering the upper plate to a gap of 1,500 µm;
- setting the temperature to the start temperature of 120°C, the constant measurement strain to 1%, the constant oscillation frequency to 1 Hz and the cooling rate to 6°C/min (from 120°C to -10°C);
- after completing the test, melting the adhesive, lifting the upper plate and removing the adhesive from both the Peltier plate and the upper plate; and
- setting the temperature back to room temperature.

Result: Tx was measured by measuring the crossover temperature between storage modulus and loss modulus for temperature higher than 50 °C. Tg was measured by measuring the temperature at the maximum of loss factor for temperature lower than 50 °C.

### Example 1

The compositions A to J were prepared by mixing the styrene block copolymer, the plasticizer and the antioxidant and then heating the mixture at 135°C. The resin was then added and after obtaining a homogenous mixture the resulting mixture was cooled at room temperature.

The materials used were the following:
- Hyprene L500: a hydrotreated naphthenic process oil commercialized by Ergon;
- Pionier^{®} TP130S: a hydrogenated esterified soybean oil commercialized by H&R and having a viscosity of 900 mPa.s at 25°C, an acidic number of 0.21 mg KOH/g and a renewable carbon content of 100%;
- D20-03: a castor oil oligoester provided by VANDEPUTTE;
- RADIA 7916: a methyl palmitate plasticizer commercialized by OLEON having a viscosity of 60 mPa.s at 25°C and a renewable carbon content of 88%;
- RADIA 7195: an isopropyl stearate plasticizer commercialized by OLEON having a viscosity of 35 mPa.s at 25°C and a renewable carbon content of 92%;
- Veopol^{®} 215035: a soybean stand oil commercialized by VandePutte having a viscosity of 35000 mPa.s at 20°C, an acidic number of 7.52 mg KOH/g and a renewable carbon content of 100%;
- Foral^{®} DX: a fully hydrogenated rosin resin commercialized by DRT having a Ring and Boll softening point around 70°C and a renewable carbon content of 100%;
- Foral^{®} 105: a fully hydrogenated rosin ester commercialized by DRT having a Ring and Boll softening point from 95 to 103°C and a renewable carbon content of 64%;
- Escorez 5400: a hydrogenated dicyclopentadiene resin commercialized by Exxon and having a renewable carbon content of 0%;
- Dercolyte^{®} M105: a polyterpene resin commercialized by DRT resulting from the polymerization of α-pinene and β-pinene and having a Ring and Ball softening point of 105°C;
- Sinopec^{®} 1126: a linear SIS copolymer commercialized by SINOPEC, having a styrene content of 16 % and a diblock content of 50%;
- Sinopec^{®} 1209: a linear SIS copolymer commercialized by SINOPEC, having a styrene content of 30 % and a diblock content of less than 1%;
- Irganox 1010: a phenolic type antioxidant commercialized by BASF.

Compositions A to F in the table below are compositions according to the invention while compositions G to J are comparative compositions.

| | A | B | C | D | E | F | G | H | I | J |
|---|---|---|---|---|---|---|---|---|---|---|
| Hyprene L500 (%) | - | - | - | - | - | - | 22 | - | - | - |
| Pionier TP130S (%) | - | 16.5 | - | - | - | - | - | 11 | 11 | - |
| D20-03 (%) | 20.9 | - | - | - | 7.6 | - | - | - | - | 20.9 |
| RADIA 7916 (%) | - | - | - | 20.9 | - | - | | - | - | - |
| RADIA 7195 (%) | - | - | - | - | - | 13.3 | - | - | - | - |
| Veopol 215035 (%) | - | - | 20.9 | - | - | - | - | - | - | - |
| Foral DX (%) | - | - | - | - | 69.5 | - | - | 62 | 31 | - |
| Foral 105 (%) | - | - | - | - | - | - | - | - | - | 56.2 |
| Escorez 5400 (%) | - | - | - | - | - | - | 56.5 | - | - | - |
| Dercolyte M105 (%) | 56.2 | 59.3 | 56.2 | 56.2 | - | 64.2 | - | - | 31 | - |
| Sinopec 1126 (%) | 6.4 | 6.8 | 6.4 | 6.4 | 6.4 | 6 | 6 | 7.6 | 7.6 | 6.4 |
| Sinopec 1209 (%) | 15.5 | 16.4 | 15.5 | 15.5 | 15.5 | 15.6 | 14.5 | 18.4 | 18.4 | 15.5 |
| Irganox 1010 (%) | 1 | 1 | 1 | 0.9 | 0.9 | 0.9 | 1 | 1 | 1 | 0.9 |
| Tg (°C) | 15 | 3.1 | 20.5 | 2.5 | 8.4 | 6.7 | 9.8 | -7.5 | -2.1 | -2.5 |

Each composition was heated at a temperature of 150°C and then coated on a polyethylene film having a width of 20 cm, at a coating weight of 3 g/m² by using a Coater CTL 4400 sold by NORDSON.

A nonwoven sheet composed of polypropylene fibers having a width of 20 cm was then put in contact with the coated surface of the polyethylene film by using a nip roll applying a pressure of 1 bar.

The laminate was then packaged as a reel and left for 24 hours at ambient temperature and at 50 % humidity.

| | A | B | C | D | E | F | G | H | I | J |
|---|---|---|---|---|---|---|---|---|---|---|
| Biocarbon content (%) | 77 | 76 | 77 | 76 | 77 | 76 | 0 | 73 | 73 | 74 |
| Tx (°C) | 100.5 | 90 | 100.4 | 58.1 | 65.6 | 83.7 | 74.4 | 68.9 | 77.8 | 77.1 |
| Viscosity (mPa.s) | 3338 | 2780 | 2795 | 917 | 2252 | 2235 | 2500 | 2181 | 2640 | 2973 |
| Average Peel (N/25 mm) | 1.6 | 1.4 | 1.3 | 2.0 | 1.5 | 1.8 | 1.6 | 0.9 | 0.7 | 0.9 |

From the above results, it is clear that the present invention makes it possible to achieve at the same time compositions that have a high biocarbon content and good peel performances (compositions A to F). Regarding the comparative compositions, on the one hand composition G shows good peel value (higher than 1 N/25 mm) but has a 0 % biocarbon content, while compositions H to J (which have a glass transition temperature lower than 0°C) have a high biocarbon content but do not have sufficiently good peel performances (lower than 1 N/25 mm).

## Claims

1. A hot melt adhesive composition comprising:
- at least one styrene block copolymer;
- at least one bio-based tackifier resin; and
- optionally at least one plasticizer chosen from:
- an esterified fatty acid; and
- a stand oil;
wherein the hot melt adhesive composition has a glass transition temperature higher than 0°C.

2. The composition according to claim 1, wherein the styrene block copolymer is chosen from a styrene-isoprene-styrene triblock copolymer and/or a styrene-butadiene-styrene triblock copolymer.

3. The composition according to claims 1 or 2, wherein the styrene block copolymer comprises at least one polydiene block which is fully or partly made from renewable materials, and is preferably chosen from a polybutadiene block, a polyfarnesene block and a polymyrcene block.

4. The composition according any one of claims 1 to 3, wherein the tackifier resin is chosen from a terpene derivative and/or a rosin derivative.

5. The composition according to any one of claims 1 to 4, wherein the tackifier resin is a terpene derivative chosen from a polyterpene homopolymer, a copolymer of terpene with a diene monomer, and a phenolic-modified terpene resin.

6. The composition according to any one of claims 1 to 5, wherein the tackifier resin is a rosin derivative chosen from a rosin and a rosin ester, preferably from a fully hydrogenated rosin and a fully hydrogenated rosin ester, and more preferably wherein the rosin derivative is a fully hydrogenated rosin ester.

7. The composition according to any one of claims 1 to 6, wherein the esterified fatty acid results from a transesterification reaction between a triglyceride and a mono-alcohol or a polyol.

8. The composition according to any one of claims 1 to 7, wherein the fatty acid comprises from 6 to 30 carbon atoms and is chosen from a hydrogenated or a non-hydrogenated fatty acid.

9. The composition according to any one of claims 1 to 8, wherein the stand oil of vegetable oil is obtained by heating said vegetable oil in the absence of oxygen and at a temperature higher than 200°C, and preferably higher than 270°C.

10. The composition according to any one of claims 1 to 9, wherein the stand oil is a stand oil of a vegetable oil comprising fatty acids containing from 6 to 30 carbon atoms.

11. The composition according to any one of claims 1 to 10, having a biocarbon content equal to or higher than 70 %.

12. The composition according to any one of claims 1 to 11, comprising from 10 to 40 % by weight of the at least one styrene block copolymer relative to the total weight of the composition, and/or from 20 to 80 % by weight of the at least one tackifier resin relative to the total weight of the composition, and/or from 0 to 25 % by weight of the at least one plasticizer relative to the total weight of the composition.

13. The composition according to any one of claims 1 to 12, wherein the tackifier resin has a Ring and Ball softening point from 70 to 150°C.

14. Use of the hot-melt adhesive composition according to any one of claims 1 to 13, for bonding two substrates.

15. An article comprising the hot melt adhesive composition according to any one of claims 1 to 13.

16. The article according to claim 15, which is a disposable absorbent article chosen from disposable diapers, adult incontinence products, sanitary napkins, medical dressings, bandages, surgical pads, and pet training pads.
